# EUROPEAN PATENT SPECIFICATION

(11) **EP 1 605 819 B1**
(45) Date of publication and mention of the grant of the patent: **09.05.2007**
(21) Application number: 04720690.9
(22) Date of filing: 15.03.2004
(51) Int. Cl.: A61B 5/00, G01N 21/65

(54) **ANALYSIS OF A COMPOSITION WITH MONITORING**
ANALYSE EINER ZUSAMMENSTELLUNG MIT BEOBACHTUNG
ANALYSE ET SURVEILLANCE D'UNE COMPOSITION

(30) Priority: 18.03.2003 EP 03100689
(43) Date of publication of application: 21.12.2005
(73) Proprietor: Koninklijke Philips Electronics N.V., 5621 BA Eindhoven (NL)
(72) Inventor: HENDRIKS, Robert, F., M., NL-5656 AA Eindhoven (NL); LUCASSEN, Gerhardus, W., NL-5656 AA Eindhoven (NL); VAN DER VOORT, Marjolein, NL-5656 AA Eindhoven (NL); PUPPELS, Gerwin, J., NL-5656 AA Eindhoven (NL); VAN BEEK, Michael, C., NL-5656 AA Eindhoven (NL)
(74) Representative: Landousy, Christian
(86) International application number: PCT/IB2004/050251
(87) International publication number: WO 2004/082474

(56) References cited:
- WO-A-01/95795
- WO-A-98/44839
- US-A- 5 814 820
- US-A- 5 865 738
- US-A- 5 865 754

## Description

The invention pertains to an analysis apparatus comprising
- an excitation system for emitting an excitation beam to excite a target region,
- a monitoring system to image the target region.

Such an analysis apparatus is known from the international application **WO02/057759.**

The known analysis apparatus employs the monitoring system to monitor the target region while the target region is excited by the excitation beam. On the basis of an image formed of the target region, the excitation beam is accurately aimed at the target region. Hence, the scattered radiation is essentially generated in the target region so that the scattered radiation that is detected includes information that essentially pertains to the material composition in the target region. Notably, the target area may be a capillary blood vessel that is located under the surface of the skin of a patient to be examined. The known analysis apparatus includes a complex and expensive confocal optical system to image the target area and receive scattered radiation from essentially only the target area below the surface of the object.

An object of the invention is to provide an analysis apparatus which is provided with a simple but yet reliable monitoring system that is less expensive to manufacture.

This object is achieved by an analysis apparatus according to the invention in which the monitoring system being arranged to produce a contrast image in a contrast wavelength range and produce a reference image in a reference wavelength range.

The contrast image and the reference image formed by the monitoring system provide additional information on the location of the target area. An insight of the invention is that in practice the target region may have low contrast to its surroundings in a particular wavelength range, but that the contrast is dependent on the wavelength range. Although the contrast may be weak, it appears more sensitive for the wavelength range. That is, although the contrast is weak, there are relatively large differences between the contrast in the contrast wavelength range and in the reference wavelength range. Accordingly, on the basis of the change of the contrast of the target region with wavelength, the target region is more reliably and more accurately identified. The difference between contrast of the target region relative to its surrounding in both contrast and reference image accurately identifies the target region. In particular when the target region concerns a blood vessel, the difference between contrast of the imaged blood vessel in the reference image having a wavelength range above 600nm, notably in the range of 600-700nm, the reference wavelength range, and contrast of the imaged blood vessel in the contrast image having a wavelength range for example the range of wavelengths for example of 520-580nm, the contrast wavelength range, is more sensitive for the blood vessel to be monitored than the contrast either in the contrast image or in the reference image. Notably, the difference in contrast between the contrast image and the reference image occurs because in the reference wavelength range blood and surrounding J tissue have almost equal absorption, whereas in the contrast wavelength range the absorption by blood is stronger than the absorption by the surrounding tissue. The differences between the contrast image and the reference image may be directly observed by the user, or these differences may be derived by a comparison system.

The monitoring system of the analysis apparatus of the invention is simpler than the confocal optical system of the known analysis apparatus and the monitoring of the target region on the basis of the comparison of the contrast image and the reference image appears to be quite reliable.

These and other aspects of the invention will be further elaborated with reference to the embodiments defined in the dependent Claims.

A preferred embodiment of the analysis apparatus of the invention comprises a comparison system to make a comparison of the contrast image with the reference image. As explained, on the basis of the comparison of the contrast image with the reference image, the target region is accurately and reliably identified. The comparison can for example be made by forming a correlation image from the contrast and reference images. The correlation image has pixel-values which represent in positions in the correlation image the correlation of the contrast image with the reference image. This correlation image involves a comparison of brightness values at corresponding positions in the contrast image and the reference image, respectively. The correlation provides a value to a quantitative relationship between pixels in the contrast image and in the reference image. A specific version of the correlation image has pixel-values that are computed as the ratio of the pixel-values of the contrast image and the reference image in corresponding positions in these images. The target region is easily identified in the correlation image as an area having pixel values that strongly deviate from the average pixel-value over the correlation image. For example, the target region may have quite high pixel-values, i.e. showing up quite brightly or quite low pixel-values, i.e. showing up as markedly dark portions in the correlation image. Good results are notably obtained when the threshold value is set to the background noise level of the correlation image. Pixel-values of the correlation image which are less than the threshold value are for example set to the digital pixel value 0. In the event that the target region shows up as dark, the target region is even more clearly identified when in the correlation image pixel-values are set to a preselected maximum (e.g. digital pixel value 255) value in positions where in the corresponding positions in the reference image the pixel-values exceed a pre-set ceiling value.

In an other version of this preferred embodiment, the comparison is made from a difference image. The difference image has pixel-values which represent in positions in the difference image the difference between the pixel-values of the contrast image and the reference image at corresponding positions. That is, the pixel-values of the difference image are computed as the differences between the pixel-values of the contrast image and the reference image in corresponding positions in these images.

In a further preferred embodiment of the analysis apparatus of the invention the monitoring system includes an optical source which produces an optical beam having a broad wavelength range. This broad wavelength range encompasses both the reference wavelength range and the contrast wavelength range. Further, the monitoring system is provided with an adjustable optical filter. A suitable adjustable optical filter is for example a liquid crystal tuneable filter (LCTF). Other examples of the adjustable optical filter are an opto-acoustic tuneable filter or a rotatable filterwheel hat has individual sections that transmit the reference wavelength range and the contrast wavelength range, respectively. In its reference setting, the adjustable optical filter allows a component of the optical beam having wavelengths in the reference wavelength range to pass, while notably wavelengths in the contrast wavelength in the optical beam are suppressed. In its contrast setting, the adjustable optical filter allows a component of the optical beam having wavelengths in the contrast wavelength range to pass, while notably wavelengths in the reference wavelength in the optical beam are suppressed. Accordingly, in the contrast setting the adjustable optical filter supplies a contrast beam having a wavelength in the contrast wavelength range. In the reference setting, the adjustable optical filter supplies a reference beam having a wavelength in the reference wavelength range. These contrast beam and reference beam are employed to form the contrast image and the reference image, respectively.

In an alternative embodiment, the monitoring system is fitted with separate optical sources that supply the contrast beam and the reference beam, respectively, so as to form the contrast image and the reference image, respectively.

In yet al alternative embodiment, the monitoring system is provided with an optical source of which the wavelength of the optical beam can be set to the reference wavelength range and to the contrast wavelength range respectively. A suitable optical source with an adjustable wavelength is an electroluminescent device which is mentioned per se in the article *'Electroluminscent device with reversible switching between red and green emission'* by S. Welter et al in Nature **421**(2003)54-57.

These and other aspects of the invention will be elucidated with reference to the embodiments described hereinafter and with reference to the accompanying drawing wherein
Figure 1 shows a schematic representation of an embodiment the analysis apparatus according to the invention,
Figure 2 shows a schematic representation of another embodiment of the invention.

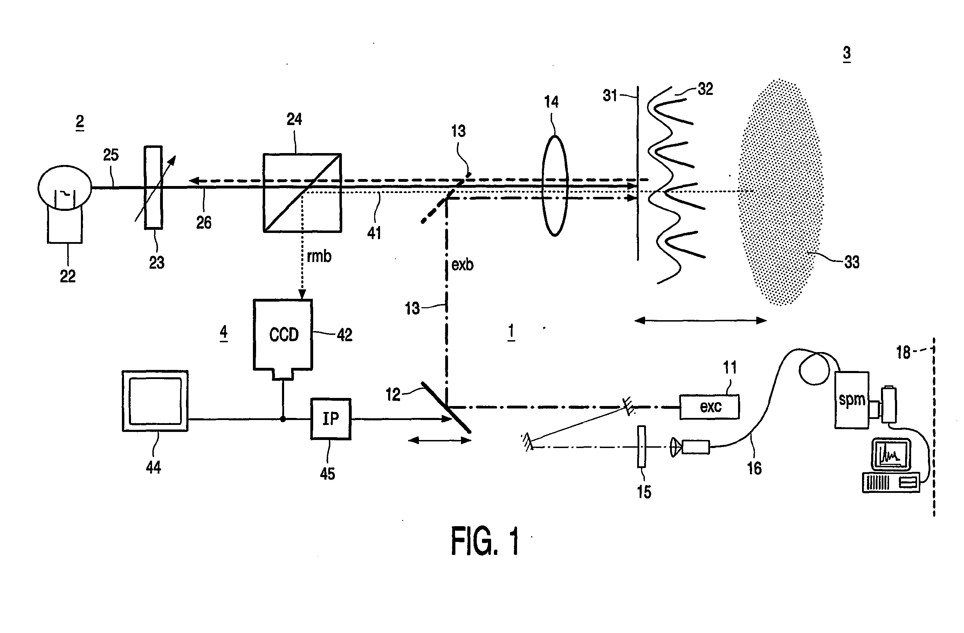

Figure 1 shows a schematic representation of the analysis apparatus according to the invention. The excitation system 1 includes an excitation laser system 11, which emits the excitation beam 13. For Raman spectroscopy, an excitation beam having a wavelength in the near-infrared range, e.g. above 800nm is often well suited. Suitable laser for the excitation laser system are for example a Titanium-Sapphire laser or a laser diode that operates at 785nm. The excitation system is also provided with a scan mirror 12, a deflecting polarising colour filter 23 and an objective lens 14 which co-operate to focus the excitation beam in the object 3 to be examined. For example, the object concerns the skin of a patient to be examined. Below the surface 31 of the skin 3, there is a layer in which capillary blood vessels 32 are located. These capillary bloodvessels occur at about 50-180µm and especially in the range of 100µm ±30µm, below the skin surface. More deeply below the surface 31, for example at about 200µm below the skin surface, there are further deep tissue layers. The excitation beam 13 is focussed on one of the capillary vessels 32 so as to generate Raman scattered radiation from the capillary vessel at issue. Thus, the capillary vessel 32 at issue forms the target region that is excited by the excitation beam 13. The Raman scattered beam 41 is supplied to a spectroscopic detection system 4 that includes a spectrometer with a CCD-camera. This spectroscopic detection system performs a spectroscopic analysis of the Raman scattered radiation from the target region, i.e. the capillary bloodvessel. Subsequently, from the acquired Raman spectral data, the constitution of the blood in the capillary bloodvessel is derived .Thus, an *in vivo* blood analysis is performed in a non-invasive manner. For example, the blood glucose level of the blood in the capillary blood vessel is determined on the basis of the Raman spectral data acquired from the blood in the capillary blood vessel. Other examples of analytes that can be examined on the basis of Raman spectroscopy are e.g. glucohemoglobin, lactate, cholesterol (total, HDL, LDL), triglycerides, hemoglobin, bicarbonate and blood gasses. The spectrometer with the CCD detector are incorporated in the spectroscopic detection system 4 which records the Raman spectrum for wavelengths that are smaller than approximately 1050nm. This upper limit of 1050nm is mainly determined by the sensitivity of the CCD-detector. The output signal of the spectrometer with the CCD detector represents the Raman spectrum of the Raman scattered infrared light. In practice this Raman spectrum occurs in the wavelength range beyond 785nm, depending on the excitation wavelength. Raman scattered infrared light has a wavelength corresponding with an energy shift in the range of 300-2000 wavenumbers relative to the excitation wavelength. The signal output of the CCD detector is connected to a spectrum display unit (spd), for example a workstation which displays the recorded Raman spectrum (spct) on a monitor.

The monitoring system 2 of the analysis apparatus is arranged to image the target region, notably the capillary blood vessel 32. A light source 22 emits an imaging beam 25 that is passed through a polariser 23 to form a polarised imaging beam 26 that is sent to the object 3, notably into the skin of the patient to be examined. To this end the polarised imaging beam has a wavelength in a range of 400-1000nm. The imaging beam preferably has a mean free scattering length in skin tissue that is larger than the depth of the capillary vessels below the skin surface, while some degree of scattering is needed to back-illuminate the capillary vessels. Adequate penetration occurs when the wavelength is larger than 400nmSuch scattering occurs increasingly as the wavelength is longer until about 850nm.. That is, good illumination of the capillary vessels is achieved in the wavelength range of 400-850nm. In order to achieve contrast between the capillary vessels and the surrounding tissue an appreciable difference between absorption by blood and tissue respectively is needed; very good results are obtained in the ranges around 520nm, 580nm and als around 430nm. Specifically, a wavelength range of preferably 520-580nm is used for the imaging beam to form the contrast image of the capillary blood vessels and for the penetration depth for the skin structure is substantial. Good results are achieved in particular at wavelengths around 530nm and in the range 560-570nm. This is yet further optimised in that the wavelength actually used is adopted to the patient to be examined at issue. Further, a wavelength range of preferably 600-700nm is employed for the imaging beam to form the reference image. Good results are for example achieved for wavelengths in the range of 620-630nm and around 630nm. After scattering and reflection from the object 3, a returning imaging beam arrives at a polarising beam splitter. The polarisation direction of the polariser 23 and the polarisation directions of the polarised beam splitter are essentially orthogonal. Only the portion of the returning imaging beam 41 of which the polarisation is essentially orthogonal to the polarisation direction of the polarised imaging beam 26 formed by the polariser 23 passes the polarising beam splitter. Thus, the polarising beam splitter acts as an analyser which essentially transmits depolarised light from the object 3 to a CCD-camera 42 of the monitoring system 4. preferably, the CCD-camera 42 is a colour-sensitive camera that is sensitive for both the reference wavelength ranges and the contrast wavelength range. Light that is reflected from the top layers of the object is hardly depolarised, while light that is scattered from deeper layers is to a larger extent depolarised. Thus, in effect, the depolarised light from the deeper layers illuminates the layers more closely to the surface. In particular, the depolarised light that is formed in the deeper layers back-illuminates the layer of capillary bloodvessels in the skin of the patient to be examined. This layer of capillary bloodvessels is located more closely to the surface of the skin than the region form which the light is scattered multiply and depolarisation occurs. Accordingly, the depolarised light functions as a back-illumination of the capillary bloodvessels. The depolarised light is employed to image the capillary vessels on the CCD-camera 41 of the monitoring system 4. In particular, back-illumination by multiple scattered light is done in two (or more) wavelength ranges. In particular, back-illumination at three separate wavelength ranges may be employed in that the reference image if formed at about 700nm and two contrast images at about 530nm and at 580nm, respectively. From a comparison of both contrast images with the reference images the oxygenation/deoxigenation ratio is determined. Accordingly, when three wavelengths are employed, spectral information in the two contrast images is employed to monitor the capillary bloodvessels and the target region, i.e. the capillary bloodvessels, is detected with a high reliability. This ratio clearly distinguishes blood in the bloodvessels from other structures that generate contrast in the contrast images. In the embodiment of the analysis apparatus of the invention shown in Figure 1, in the polariser 23 there is integrated the adjustable optical filter. This is achieved in that there is employed an adjustable polarising colour filter 23, that transmits essentially one polarisation direction of a component in respective wavelength ranges of the light from the light source 22. Notably, when transmission window of the adjustable polarising colour filter is set to the contrast wavelength range of for example 520-580nm so as to achieve the formation of the contrast image, which is practise is a predominantly green image in which bloodvessels are imaged with a substantial contrast. When the transmission window of the adjustable polarising colour filter is set to the reference wavelength range of for example 600-700nm, the reference image is formed, which in practice is a predominantly red image in which especially bloodvessels are image at low contrast to its surroundings. There occurs, however some contrast in the reference image occurs notably due to anatomical structures other than bloodvessels. Namely, some contrast may be generated for example by structured tissue fibres that depolarise or are birefrigent, notably, grooves, sweat glands or hair follicles.

The CCD-camera 42 outputs respective electronic image signals, namely a contrast image signal and a reference image signal that represent the contrast image and reference image, respectively. Notably signal levels of the contrast and reference image signal correspond to the brightness values of the contrast and reference images respectively. The contrast and reference image signals are applied to a monitor 43 on which the contrast image and the reference image are displayed. Preferably, the contrast image and the reference image are displayed simultaneously through respective view ports on the monitor 44. Further, the contrast image signal and the reference image signal are applied to an image processing unit 45. The image processing unit is programmed to compare the contrast image signal with the reference image signal. This comparison provides information on the location of the target region, notably a capillary blood vessel just beneath the skin surface of the patient to be examined. On the basis of this comparison of the contrast image with the reference image, the image processing unit controls the excitation system 1 so as to excite the capillary blood vessel that is detected in the comparison of the contrast image with the reference image. Notably, the image processing unit adjusts a scan mirror 12 of the excitation system so as to accurately direct the excitation beam 13 to the capillary blood vessel at issue. Alternatively, the capillary blood vessel may be positioned accurately with respect to the excitation beam. For example, an x.y-table may be used to support for example the patient's hand or wrist so that the patient's hand or wrist may be moved in order to correctly place the capillary vessel.

The image processing unit is for example programmed to compute the correlation of the contrast image to the reference image. High values of this correlation indicate the presence of a blood vessel with high reliability and high accuracy. In another version, the image processing unit is for example programmed to compute the ratio of brightness values in corresponding positions in the contrast image and the reference image, respectively. This can simply be done by computing the ratio of the signal level of the contrast image signal to the signal level of the reference image for corresponding positions in both contrast and reference images. Corresponding positions in both images relate to essentially the same position in the object 3.

The excitation system includes an excitation laser 11 to produce an excitation laser beam which for example emits infrared light in the range of 700-850nm in particular around 785nmat a bandwidth of about 0.1nm. This excitation beam generates Raman scatter light that returns from the patient's skin to a fibre entrance 17 of a fibre coupling 16 to the spectroscopic detection system 18. A filter 15 is placed in front of the fibre entrance 17. The filter 15 essentially blocks elastically scattered or reflected laser light having the same wavelength as the excitation beam. Hence, the filter 15 avoids that part of the excitation beam is entered into the fibre 16. The spectroscopic detection system performs a spectroscopic analysis of the contents of the capillary bloodvessel at issue. Because the excitation beam has been accurately aimed at this bloodvessel based on the correlation of the information in the contrast and reference images, corruption of the spectroscopic analysis by Raman scattering from beyond the capillary bloodvessel at issue is substantially avoided.

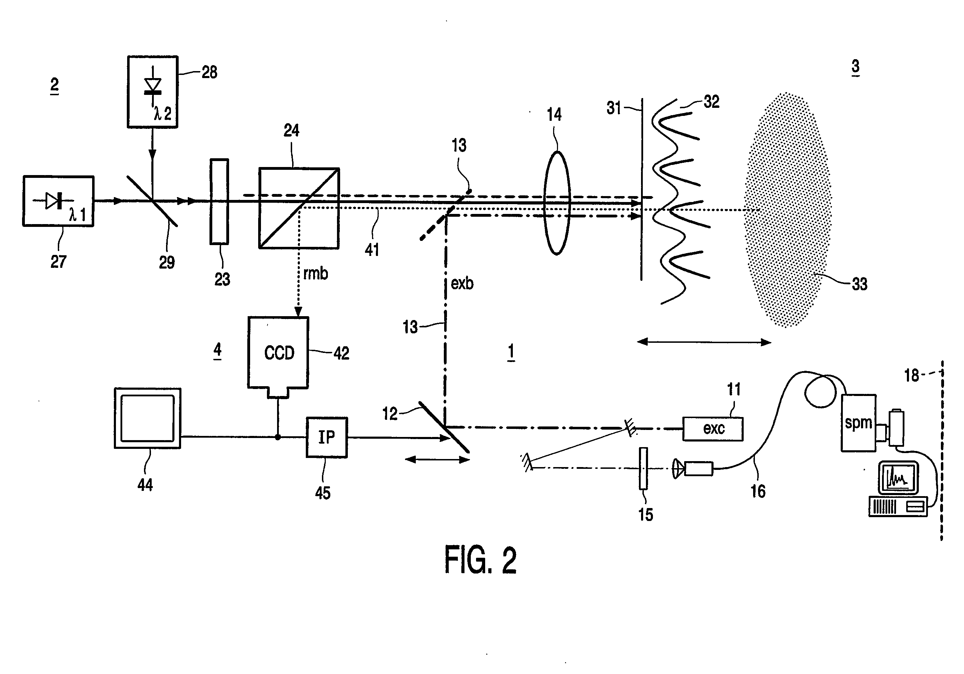

Figure 2 shows a schematic representation of another embodiment of the invention. In the embodiment shown in Figure 2, the monitoring system 2 comprises two diode lasers or LEDs 27, 28. These are a reference laser diode 27 and a contrast laser diode 28 One of the injection diode lasers, the reference laser diode 27 emits light in the reference wavelength range and the other laser diode, the contrast laser diode 28 emits light in the contrast wavelength range. Notably, the reference laser diode emits red light having a wavelength of about 630nm and the contrast laser diode emits green light of about 530nm. In stead of laser diodes, light emitting diodes (LEDs) may be employed. The monitoring system further includes a selective reflector 29. The selective reflector 29 has a high transmissivity for the reference beam from the reference laser diode 27, on the other hand the selective reflector 29 has a reflectivity for the contrast beam from the contrast laser diode 28. The CCD-camera 42 is operated such that it acquires the reference image and the contrast image successively.

## Claims

1. An analysis apparatus, in particular a spectroscopic analysis apparatus, comprising
- an excitation system for emitting an excitation beam to excite a target region,
- a monitoring system to image the target region, the monitoring system being arranged to
- produce a reference image obtained by illumination of an imaged region covering the target region at a reference wavelength range, and
- produce a contrast image obtained by illumination of the imaged region at a contrast wavelength range for which the relative contrast of the target region with respect to its surrounding is greater than the relative contrast of the target region with respect to its surrounding for the reference wavelength range,
- a comparison system for identifying the target region based on a comparison of the reference image and contrast image.

2. An analysis apparatus as claimed in Claim 1, wherein the comparison system is configured to
- derive a correlation image from the contrast image and the reference image or to
- derive a subtracted image from the contrast image and the reference image.

3. An analysis apparatus as claimed in Claim 1, wherein the monitoring system is provided with
- an optical source to produce an optical beam having a broad wavelength range that covers at least the contrast wavelength range and the reference wavelength range,
- an adjustable optical filter system having an adjustable passband and the adjustable optical filter having
- a reference setting in which the adjustable passband has support within the reference wavelength range and
- a contrast setting in which the adjustable passband has support within the contrast wavelength range.

4. An analysis apparatus as claimed in Claim 3, wherein the adjustable filter system has a passband which includes a wavelength range of scattered radiation generated by the excitation beam from in the target region.

5. An analysis apparatus as claimed in Claim 1, wherein the monitoring system is provided with an optical source to produce an optical beam having a wavelength range that is adjustable to be set to the reference wavelength range and to the contrast wavelength range.

6. An analysis apparatus as claimed in Claim 1, wherein
- the contrast wavelength range is included in an interval around 580nm
- the reference wavelength range is included in an interval around 680nm.

7. An analysis apparatus as claimed in Claim 1, wherein the monitoring system is an orthogonal polarisation spectral imaging system.

## Patentansprüche

1. Analysegerät, insbesondere spektroskopisches Analysegerät, mit
- einem Anregungssystem zum Emittieren eines Anregungsstrahlenbündels für die Anregung einer Zielregion;
- einem Beobachtungssystem zur Darstellung der Zielregion, wobei das Beobachtungssystem so ausgelegt ist,
- dass es ein Referenzbild erzeugt, welches durch Beleuchtung einer abgebildeten Region, die die Zielregion bei einer Referenzwellenlänge abdeckt, erreicht wird, und
- dass es ein Kontrastbild erzeugt, welches durch Beleuchtung der abgebildeten Region bei einem Kontrastwellenlängenbereich erreicht wird, bei dem der relative Kontrast der Zielregion in Bezug auf ihre Umgebung größer ist als der relative Kontrast der Zielregion in Bezug auf ihre Umgebung im Referenzwellenlängenbereich.
- ein Vergleichssystem zum Identifizieren der Zielregion basierend auf einem Vergleich des Referenzbildes mit dem Kontrastbild.

2. Analysegerät nach Anspruch 1, wobei das Vergleichssystem konfiguriert ist, um
- ein Korrelationsbild von dem Kontrastbild und dem Referenzbild abzuleiten, oder um
- ein subtrahiertes Bild von dem Kontrastbild und dem Referenzbild abzuleiten.

3. Analysegerät nach Anspruch 1, wobei das Beobachtungssystem ausgestattet ist mit
- einer optischen Quelle zum Erzeugen eines optischen Strahlenbündels mit einem breiten Wellenlängenbereich, der mindestens den Kontrastwellenlängenbereich und den Referenzwellenlängenbereich abdeckt,
- einem justierbaren optischen Filtersystem mit einem justierbaren Durchlassbereich und wobei der justierbare optische Filter
- eine Referenzeinstellung hat, in der der justierbare Durchlassbereich innerhalb des Referenzwellenlängenbereichs unterstützt wird und
- eine Kontrasteinstellung hat, in der der justierbare Durchlassbereich innerhalb des Kontrastwellenlängenbereichs unterstützt wird.

4. Analysegerät nach Anspruch 3, wobei das justierbare Filtersystem einen Durchlassbereich hat, der einen Wellenlängenbereich von Streustrahlung umfasst, welche durch das Anregungsstrahlenbündel von der Zielregion erzeugt wurde.

5. Analysegerät nach Anspruch 1, wobei das Beobachtungssystem mit einer optischen Quelle ausgestattet ist, um ein optisches Strahlenbündel mit einem Wellenlängenbereich zu erzeugen, der justierbar ist, um auf den Referenzwellenlängenbereich und auf den Kontrastwellenlängenbereich eingestellt zu werden.

6. Analysegerät nach Anspruch 1, wobei
- der Kontrastwellenlängenbereich in einem Intervall um 580 nm enthalten ist,
- der Referenzwellenlängenbereich in einem Intervall um 680 nm enthalten ist.

7. Analysegerät nach Anspruch 1, wobei das Beobachtungssystem ein Orthogonalpolarisations-Spektralbildgebungssystem ist.

## Revendications

1. Appareil d'analyse, en particulier un appareil d'analyse spectroscopique, comprenant :
- un système d'excitation pour émettre un faisceau d'excitation pour exciter une zone cible ;
- un système de surveillance pour représenter la zone cible par une image, le système de surveillance étant à même de
- produire une image de référence obtenue par l'éclairement d'une zone représentée par une image couvrant la zone cible à une gamme de longueurs d'onde de référence, et
- produire une image de contraste obtenue par l'éclairement de la zone représentée par une image à une gamme de longueurs d'onde de contraste pour laquelle le contraste relatif de la zone cible par rapport à son environnement est supérieur au contraste relatif de la zone cible par rapport à son environnement pour la gamme de longueurs d'onde de référence ;
- un système de comparaison pour identifier la zone cible sur la base d'une comparaison de l'image de référence et de l'image de contraste.

2. Appareil d'analyse suivant la revendication 1, dans lequel le système de comparaison est configuré pour
- dériver une image de corrélation de l'image de contraste et de l'image de référence ou pour
- dériver une image soustraite de l'image de contraste et de l'image de référence.

3. Appareil d'analyse suivant la revendication 1, dans lequel le système de surveillance est pourvu de
- une source optique pour produire un faisceau optique présentant une large gamme de longueurs d'onde qui couvre au moins la gamme de longueurs d'onde de contraste et la gamme de longueurs d'onde de référence ;
- un système de filtre optique réglable présentant une bande passante réglable et le filtre optique réglable comportant
- un paramétrage de référence dans lequel la bande passante réglable est supportée par la gamme de longueurs d'onde de référence, et
- un paramétrage de contraste dans lequel la bande passante réglable est supportée par la gamme de longueurs d'onde de contraste.

4. Appareil d'analyse suivant la revendication 3, dans lequel le système de filtre réglable présente une bande passante qui inclut une gamme de longueurs d'onde de rayonnement dispersé généré par le faisceau d'excitation depuis l'intérieur de la zone cible.

5. Appareil d'analyse suivant la revendication 1, dans lequel le système de surveillance est pourvu d'une source optique pour produire un faisceau optique présentant une gamme de longueurs d'onde qui est réglable pour être fixée sur la gamme de longueurs d'onde de référence et sur la gamme de longueurs d'onde de contraste.

6. Appareil d'analyse suivant la revendication 1, dans lequel
- la gamme de longueurs d'onde de contraste est incluse dans un intervalle autour de 580 nm ;
- la gamme de longueurs d'onde de référence est incluse dans un intervalle autour de 680 nm.

7. Appareil d'analyse suivant la revendication 1, dans lequel le système de surveillance est un système d'imagerie spectrale à polarisation orthogonale.
